# EUROPEAN PATENT APPLICATION

(11) **EP 2 589 657 A1**
(43) Date of publication of application: **08.05.2013**
(21) Application number: 11801003.2
(22) Date of filing: 01.07.2011
(51) Int. Cl.: C12N 15/09, C12Q 1/68

(54) **METHOD FOR DETECTION OF TARGET MOLECULE**

(30) Priority: 01.07.2010 JP 2010151242
(71) Applicant: National Institute of Advanced Industrial Science and Technology, Tokyo 100-8921 (JP)
(72) Inventor: MIYAGISHI Makoto, Tsukuba-shi Ibaraki 305-8566 (JP)
(74) Representative: Woods, Geoffrey Corlett
(86) International application number: PCT/JP2011/065205
(87) International publication number: WO 2012/002541

(57) **Abstract**

The present invention provides a method for detecting a target molecule using the sequence information of a collection (pool) of aptamers capable of specifically binding to a target molecule, comprising the following steps of: (a) bringing a target molecule into contact with a collection of oligonucleotides which comprise multiple nucleic acid aptamers having different randomized sequences; (b) selecting a subcollection of oligonucleotides that bind to the target molecule; (c) examining the sequence of each oligonucleotide of the selected subcollection; (d) extracting sequence information which is characteristic to the oligonucleotides having affinity for the target molecule, from the sequences of the selected oligonucleotides; and (e) identifying the target molecule based on the sequence information.

## Description

### Technical Field

The present invention relates to a novel method for detecting a target molecule using the sequence information of a collection (pool) of aptamers which enable to specifically bind to the target molecule.

### [Background Art]

Detection of proteins or the like that can be served for disease markers has become extremely important for diagnosis or treatment of various diseases. In the case of detecting a biomolecule such as a protein, a method comprising using a detection molecule that enable to specifically bind to a target molecule for detection, and detecting the target molecule using the interaction between the detection molecule and the target molecule, is generally employed. For example, with the use of an antibody interacting with an antigen as a target molecule, various methods including agglutination (such as immunonephelometry), precipitation, ELISA, and immunochromatography are used.

In recent years, many attempts for detection by use of aptamers enable to specifically binding to target molecules, as detection molecules instead of antibodies, have been conducted. An aptamer is a functionally single-stranded nucleic acid, so that it is advantageous for being conveniently synthesized using an automatic nucleic acid synthesizer or the like, and, it is less expensive than antibodies mainly composed of protein.

To detect a target molecule using an aptamer, first, an aptamer specifically binding to a target molecule should be specified.
To select and identify such an aptamer, the SELEX method (e.g., see Patent Literature 1 or 2) is known, which is a technique comprising: bringing an oligonucleotide library, which is a mixture of many oligonucleotides containing random sequences, into contact with a target molecule, selecting a collection (pool) of oligonucleotidse having high affinity for the target molecule, amplifying them, and then confirming whether or not the selected oligonucleotidse specifically binds to the target molecule.

However, to obtain a specific aptamer specifically binding to a target molecule, there is a need to prepare an oligonucleotide library comprising a huge number of oligonucleotides in order to prevent failure to detect a target aptamer, and then to repeat many times the selection and amplification of aptamers having high affinity by means of the SELEX method.

In recent years, as a modified SELEX method, a counter SELEX method (e.g., Non-patent Literature 1) or a method for identifying an aptamer specifically binding to a target molecule by repeating 5 to 10 times the step of amplifying and producing the thus selected oligonucleotide collection (pool) having high affinity and then binding it again to a target molecule, has been developed (Patent Literature 3). However, these methods require excessive effort and can not serve as techniques with which such an aptamer can be conveniently screened for.

Even if an aptamer specifically binding to a target molecule can be found, the aptamer should be labeled in advance with a fluorescent substance, a radio isotope, or the like so that it can be used for detecting the target molecule. This is because the thus labeled aptamer is bound to the target molecule, and then signals coming from the label are measured for detection. However, when an aptamer is labeled with such a label, the degree of binding between the aptamer and a binding molecule decreases due to the effect of the label, often making precise measurement difficult. Modified detection methods such as a method using a linker (e.g., Non-patent Literature 2 or 3) and a method using a nucleic acid label that forms a complex with an aptamer (Patent Literature 4) have been developed. A method for effectively labeling an aptamer has not yet been established. Furthermore, it is difficult in many cases to specifically identify a target substance with high accuracy using only a single type of aptamer. Moreover, the development of a specific detection method with even higher accuracy is thought to be required.

### Prior Art Literature

### Patent Literature

Patent Literature 1: U.S. Patent No. 5270163
Patent Literature 2: JP Patent Publication (Kokai) No. H08-252100A (1996)
Patent Literature 3: JP Patent Publication (Kokai) No. H08-252100A (1996)
Patent Literature 4: JP Patent Publication (Kokai) No. 2010-41951A

### Non-patent Literature

Non-patent Literature 1: "Frontiers of Antibody Engineering," Yoshihiro ITO, CMC Publishing CO., LTD., 2004, pp. 115-122 (Japan)
Non-patent Literature 2: Yoshihiro MIWA (Ed.,), YODOSHA, "How to Select and Use Fluorescent-Luminescent Reagent (Keiko/Hakko Shiyaku no Erabikata to Tsukaikata)," pp. 60-61 (Japan)
Non-patent Literature 3: Komatsu (Komatsu, Y.) et al., Bioorganic & Medicinal Chemistry, 2008, Vol. 16, No. 2, pp. 941-949

### Summary of the Invention

### Problem to be Solved by the Invention

As described above, what are required in a case of detecting a target molecule using an aptamer requires are the following two steps of: (1) specifying identifying an aptamer specifically binding to a target molecule; and (2) labeling the aptamer, and then causing it to bind to the target molecule, so as to detect the target molecule. However, both steps are problematic as described above and are insufficient in terms of accuracy and sensitivity.

### Means for Solving the Problem

As a result of intensive studies to address the above problems, the present inventors have now obtained the sequence information characteristic of to a collection (pool) of oligonucleotides having affinity for a target molecule by bringing a collections of oligonucleotides comprising multiple nucleic acid aptamers that have different random sequence regions into contact with the target molecule, selecting a subcollection (subpool) of oligonucleotides that have bound to the target molecule, and then examining the sequences of the subcollection of oligonucleotides. Thus, they present inventors have now found that such a target molecule can be detected using the subcollection of oligonucleotides and have completed the present invention.

Specifically, the present invention is as follows.
[1] A method for detecting a target molecule, comprising the following steps of:
   (a) bringing a target molecule into contact with a collection of oligonucleotides which comprise multiple nucleic acid aptamers having different randomized sequences;
   (b) selecting a subcollection of oligonucleotides that bind to the target molecule;
   (c) examining the sequence of each oligonucleotide of the selected subcollection;
   (d) extracting sequence information which is characteristic to the oligonucleotides having
   affinity for the target molecule, from the sequences of the selected oligonucleotides; and (e) identifying the target molecule based on the sequence information.

[2] The method according to [1] above, wherein the above collections of oligonucleotides has nucleotide sequences represented by general formula X N₁X₂ N₂...X ₙ₋₁Nₙ₋₁Xₙ (where each X is a specific nucleotide sequence having a specific length, and, at least one X may be deleted from among Xs except for X₁ and Xₙ, each N has a length of 1 nucleotide, and, is G, A, C, T or U, or a modified nucleotide thereof, and "n" is an integer ranging from 2 to 30).

[3] The method according to [2] above, wherein X₁ complementarily binds to Xₙ.

[4] The method according to any one of [1] to [3] above, wherein the number of nucleic acid aptamers in the above collection of oligonucleotides is between 3 and 10⁹, inclusive.

[5] The method according to any one of [2] to [4] above, wherein "n" in the above general formula is an integer ranging from 2 to 30.

[6] The method according to any one of [1] to [5] above, wherein the step (b) of selecting a subcollection of oligonucleotides bound to a target molecule comprises removing by washing the collections of oligonucleotides unbound to the target molecule using the immobilized target molecule or the target molecule bound to a carrier, and then selecting oligonucleotides bound to the target molecule.

[7] The method according to any one of [1] to [5] above, wherein the step (b) of selecting a subcollection of oligonucleotides bound to the target molecule comprises digesting unbound oligonucleotides using an enzyme and then selecting oligonucleotides bound to the target molecule.

[8] The method according to any one of [1] to [7] above, wherein the step (c) of examining the sequence of each oligonucleotide of the selected subcollection comprises determining the nucleotide sequence.

[9] The method according to any one of [1] to [8] above, wherein the step (d) of extracting the sequence information comprises the extraction of sequence information characteristic to the subcolleciton of oligonucleotides having affinity for the target molecule, by comparison of the sequence of subcolleciton of oligonucleotides bind to the target molecule, with the sequence of subcolleciton of oligonucleotides which is obtained with the use of a molecule other than the target or without using any target,

[10] The method according to any one of [1] to [9] above, wherein the characteristic subcollection of oligonucleotides obtained by sequencing in [8] above is used.

[11] The method according to any one of [1] to [10] above, further comprising: (f) a step of amplifying the oligonucleotides of the selected subcollection, between the steps (b) and (c).

[12] The method according to any one of [1] to [11] above, wherein the sequences of the collection of oligonucleotides partially comprise modified nucleotides.

[13] The method according to [12] above, wherein the above modification is methylation.

[14] The method according to any one of [1] to [13] above, wherein the nucleic acid aptamers in the collection of oligonucleotides are bound to a peptide, an amino acid, a sugar, a polyamine, or a lipid.

[15] The method according to any one of [1] to [14] above, wherein the nucleic acid aptamers of the collection of oligonucleotides are chimeras of DNA and RNA.

[16] A method screening for a nucleic acid aptamer specifically binding to a target molecule from the collection of oligonucleotides, characterized in that the screening is carried out by using the method according to any one of [1] to [15] above.

[17] A method for quantifying a target molecule, comprising: adding a sample containing a target molecule to a nucleic acid aptamer that does not bind to the target molecule, and, a nucleic acid aptamer that specifically binds to the target molecule; treating both nucleic acid aptamers together with nuclease; and then examining the ratio of the amounts of both nucleic acid aptamers thereby to quantify the target molecule.

[18] The method according to [17] above, wherein the target molecule is quantified using the nucleic acid aptamer specifically binding to the target molecule, which aptamer is obtained by the method of [16] above.

This description includes all or part of the contents as disclosed in the description and/or drawings of Japanese Patent Application No. 2010-151242, from which the present application claims the priority.

The present invention provides a novel method for detecting a target molecule using a collection of oligonucleotides comprising multiple nucleic acid aptamers that have different randomized sequences. The novel method does not require a step as seen in conventional methods of specifying identifying an aptamer specifically binding to a target molecule. Alternatively, unlike the SELEX method that involves repeating steps of comprising binding, fractionation (selection), elution, and amplification, so as in order to select an aptamer, the novel method does not require repetition of such a steps and can complete the selection at one time. Moreover, the novel method is advantageous in that it enables efficient high-precision detection of a target molecule using multiple nucleic acid aptamers having affinity for the target molecule. Also, this novel method has a further advantage that the method can use a collection (pool) of oligonucleotides comprising nucleic acid aptamers comprising a modified nucleotide(s), a substance(s) linked thereto, or a RNA/DNA chimera(s), which has/.have been unable to be used in the SELEX method.

The present invention further provides a method for screening for an aptamer specifically binding to a target molecule. Furthermore, the present invention provides a method for quantifying a target molecule by causing the aptamer to bind to the target molecule and then treating the resulting aptamer/target molecule complex with nuclease.

### Brief Description of the Drawings

Fig. 1 shows the results of quantifying streptavidin by gel electrophoresis using S1 nuclease and streptavidin aptamers.
Fig. 2 is a graph prepared by converting the results (numerical figures) in Fig. 1 into graph form. The horizontal axis indicates the ratio of streptavidin aptamer to control aptamer, and, the vertical axis indicates streptavidin concentration (µg/ml).

### Embodiments for Carrying Out the Invention

Hereafter, the present invention will further be specifically explained while showing the embodiments of the present invention.

### 1. Definition

Terms used herein are used according to standard meanings basically used in the fields of chemistry, life science, genetic engineering, and the like. To more clearly explain the present invention, some terms used to explain the present invention are as described below.

The term "oligonucleotide" as used herein refers to a nucleic acid molecule containing at least about 20, about 50, or about 100 nucleotides, and preferably 200 or fewer nucleotides that are bound via diester phosphate linkage. Examples of nucleotides include guanine (G), adenine (A), cytosine (C), thymine (T), and uracil (U), as well as modified nucleotides thereof (or modified bases) (where each nucleotide may be either ribonucleotide or deoxyribonucleotide).

Furthermore, the types, sizes, sequences and the like of the nucleic acids in the collection of oligonucleotides can be appropriately designed depending on the type, properties, characteristics, and the like of a target molecule binding to oligonucleotides. For example, the nucleic acids may have the structure of a single-stranded portion or the structure of a double-stranded portion, and furthermore, may have a stem-loop structure, or another stable structure, such as a bulge loop structure, a hairpin structure, a pseudoknot structure, a G quartet structure, or a triple helix structure.

The term "general formula "X₁ N₁X₂N₂ ...Xₙ₋₁Nₙ₋₁Xₙ" as used herein representing the collection of oligonucleotides refers to the a nucleotide sequences of a nucleic acid aptamers having a randomized sequence portions (N , N , ..., N), wherein "X₁, X₂, X₃, ..., Xₙ" are specific fixed nucleotide sequences (including a case in which at least one X is deleted and thus the nucleotide sequence composing the "X" is absent) having specific (or "given") lengths, "X" has appropriately a length of 1 to 30 nucleotides, for example, and particularly, X₁ and Xₙ have lengths that allow the annealing of amplification primers, such as a length ranging from 10 to 30 nucleotides. "N" is variable for randomization of oligonucleotides and has a length of 1 nucleotide. Nucleotides composing "X" and "N" may comprise any nucleotides, which (may include any of ribonucleotide, deoxynucleotide, and modified nucleoetide,) including guanine (G), adenine (A), cytosine (C), thymine (T) or uracil (U), or modified nucleoetides thereof (or artificial nucleotides) thereof. Also, "X₁" and "Xₙ" may have nucleotide sequences that complementarily bind. Herein, the term "specific" means fixing aeach sequence to a specific (or given) sequence. Such a "specific" sequence is distinguished from the randomized sequence portion. For example, in the collection of oligonucleotides, which is represented by 5'-GUUGAGN₁N₂N₃AUUACGN₄N₅CUCAG-3' (SEQ ID NO: 20), GUUGAG on the 5'-eterminal side corresponds to "X₁," and "X₄" corresponds to AUUACG, and "X₆" corresponds to CUCAG. In this example, X₂, X₃, and X₅ lack nucleotide sequences corresponding thereto, meaning that N₁ and N₂, N₂ and N₃, and N₄ and N₅ are directly linked to each other.

In the above-described nucleotide sequence, "n" indicates an integer that is, for example, an integer of between 2 and 30, and preferably an integer of between 3 and 15 (e.g., an integer of between 6 and 12), but the "n" is not limited to said range.

The term "complementarily bind" as used herein means that two nucleotide sequences (which are X₁ and Xₙ) form base pairs with each other so as to form complementary double strands. A base pair is generally formed by hydrogen bonding between A and T, A and U, C and G, or G and U. Under conditions where the formation of complementary double strands is not disturbed, a mismatch base pair(s) or a bulge base(s) may be contained. In such a case, a mismatch or bulge of 1 to 2 nucleotides may be contained per 5 nucleotides.

The term "modified nucleoetide" (or referred to as "modified base") as used herein refers to an artificial nucleotide generally differing from 4 types of natural dNTP (here "N" is G, A, C, or T) or NTP (here "N" is G, A, C, or U), in which a sugar portion, a nucleotide portion, or a diester phosphate group is entirely or partially chemically-modified. Examples of chemical modification include, but are not limited to, lower alkylation (e.g., methylation, ethylation, propylation, or the like), halogenation (fluorination, cholorination, bromination, or iodination), thiolation (-SH), phosphoro-modification (-S⁻), amination (-NH₂), amidation (-CONH₂), and acetylation (-COCH₃). Examples of such modified nucleoetides include, a 2'-O-methyl modified nucleotide, a nucleotide having phosphorothioate linkage, a 6-methyl modified nucleoetide, N,N-dimethyl adenine, 2-propyl modified nucleoetide, 2-aminoadenine, and a cross-linking nucleic acid (BNA (2',4'-Bridged Nucleic Acid; also referred to as LNA (Locked Nucleic Acid)); S. Obika et al., Tetrohedron Lett., 39: 5401-5404, 1998).

The term "nucleic acid aptamer" as used herein refers to a nucleic acid molecule having a short sequence from about 20 to about 200 nucleotides, having high affinity for a given target molecule, and being capable of specifically binding to the target molecule, for example. A nucleic acid molecule having such properties is referred to as a nucleic acid aptamer, and is not limited by nucleotide sequence, molecular size, molecular conformation, and the like, unless otherwise specified. Examples of such a nucleic acid aptamer include an RNA aptamer, a DNA aptamer, and a DNA-RNA hybrid aptamer (also referred to as a chimeric DNA/RNA aptamer).

The term "a collection (pool) of oligonucleotides comprising multiple nucleic acid aptamers that have different randomized sequences" as used herein refers to, but are not limited to, a nucleic acid aptamer oligonucleotide library, wherein the number of nucleic acid aptamers in a collection of oligonucleotides is, but is not limited to, between 3 and 10⁹ inclusive, and is preferably between 10 and 10⁷ inclusive, and different sequences, the sequence patterns of which have been randomly prepared, are contained. However, the number of nucleic acid aptamers may be the order of 10⁹ or more, for example the order of 10¹¹_{.}

The term "target molecule" as used herein refers to a molecule that is a target to be detected in a detection method using a nucleic acid aptamer. Examples of the chemical species of such a target molecule are not particularly limited and include various inorganic chemical species or organic chemical species such as low-molecular-weight compounds, high-molecular-weight compounds, and substances from living organisms. More specific examples of a target molecule include sugars, lipids, oligo peptides, proteins, and nucleic acids. Also, examples of functional species that can be a target of a target molecule include an antigen, an antibody, a ligand, a receptor, an interacting protein, and a virus. Specific examples of such a target molecule are described in R. Stoltenburg et al., Biomolecular Engineering 24: 381-403, 2007.

The expression "a target molecule binds to an oligonucleotide(s)" as used herein means that a target molecule and a specific oligonucleotide(s) are bound with selectivity or affinity. Also, specific binding of an oligonucleotide(s) to a target molecule is not limited by its binding mode, and examples thereof include chemical binding such as covalent bonding, ionic bonding, hydrogen bonding, and electrical adsorption, and physical binding such as shape-dependent conjugation.

The term "sequence information characteristic to oligonucleotides" refers to information concerning the sequences of oligonucleotides having affinity for and specificity to a target molecule.

The term "amplification" as used herein refers to the amplification of a nucleic acid clone. Examples of an amplification method include, but are not limited to, the PCR method, the LAMP method, and the SMAP method. The LAMP method (Loop-Mediated Isothermal Amplification) involves setting 4 types of primers for 6 regions of a target gene, and performing a reaction at a constant temperature (about 65°C) using a chain substitution reaction for amplification, wherein chain substitution DNA synthase is used (T. Notomi et al., Nucleic Acids Res. 28: E63 (2000)). The SMAP method (SMart Amplification Process) is an isothermal nucleic acid amplification method, which involves designing, in the nucleic acid amplification method using a chain substitution reaction, a primer that can form a stem-loop structure only when a target nucleic acid is amplified, so as to satisfy specific conditions, and using the primer and a primer having a folding sequence at the 5' end portion in combination, so as to amplify the target nucleic acid specifically and efficiently (JP Patent Publication (Kokai) No. 2008-161165 A).

### 2. Method for detecting target molecule

Next, the embodiments of the method for detecting a target molecule of the present invention are as described below. However, the present invention is not restricted to the following embodiments and various modifications can be allowed in a range within the scope thereof.

Biochemical or genetic engineering techniques in the present invention are performed with reference to various experimental manuals such as Molecular Cloning: A LABORATORY MANUAL, 3rd edition, Cold Spring Harbor Laboratory Press, Cold Spring Harbor, New York (2001), New Genetic Engineering Handbook (Shin Idenshiko-gaku Handbook, 4th revised edition, ed., Muramatsu et al., YODOSHA, Japan (2003), Protein Experimental Protocols (Tanpaku-shitsu Jikken no Susmekata) (Masato Okada, ed., Miyazaki Kaoru, YODOSHA, Japan, 1st edition, 1998), and Protein Experiment Note (Tanpakushitsu Jikken Note) (ed., Masato Okada and Kaoru Miyazaki, YODOSHA, Japan, 2nd edition, 1999).

The present invention relates to a method for detecting a target molecule having high affinity for a specific nucleic acid aptamer from a collection of oligonucleotides comprising multiple types of candidate nucleic acid aptamers. This method can also be used as a method for screening for a nucleic acid aptamer having high affinity for and specifically binding to the target molecule. Each step of the method is as specifically described below.

The method of the present invention comprises the following steps (a) to (e).

The method comprises the steps of:
(a) bringing a target molecule into contact with a collection of oligonucleotides comprising multiple nucleic acid aptamers that have different randomized sequences;
(b) selecting a subcollection of oligonucleotides binding to the above target molecule;
(c) examining each sequence of the above-selected subcollection of oligonucleotides;
(d) extracting sequence information characteristic to the subcollection of oligonucleotides having affinity for the target molecule, among the above-selected oligonucleotide sequences; and
(e) identifying the target molecule based on the above sequence information.

### 2.1. Step (a)

In this step, a target molecule is brought into contact with a collection of oligonucleotides comprising multiple nucleic acid aptamers having different randomized sequences.

The collection of oligonucleotides comprising multiple nucleic acid aptamers having different sequences can be obtained from a pool of nucleic acid sequences prepared by randomizing "N" in the above-descrobed general formula. For sequence randomization, only natural nucleotides may be randomly arranged, or alternatively, only artificial nucleotides may be randomly arranged, or alternatively, natural nucleotides and artificial nucleotides may be randomly or alternately arranged. The size of the pool in this case is determined based on the number of nucleotides (N) to be randomized. For example, to exhaustively cover many pools of nucleic acid aptamers by using a next-generation sequencer for sequencing nucleotide sequences, the number of "N" in the above-described general formula is between 1 and 29 inclusive, and preferably between 2 and 14 inclusive, such as between 6 and 12 inclusive. The number of "N" may be greater than 29 and may range from 30 to 50, for example. This is because when the number of "N" is high, "N" can be a mixture of 2 nucleotides such as G and A. In such a case, even if the number of "N" is high, the total number of pools is limited.

The number of nucleic acid aptamers in a collection of oligonucleotides is, but is not limited to, between 3 and 10⁹ inclusive, and preferably between 10 and 10⁷ inclusive. However, the number of nucleic acid aptamers may be 10⁹ or more, such as the order of 10¹¹.

The collection of oligonucleotides may contain modified nucleoetides and additionally peptides, amino acids, sugars, polyamines (e.g., spermin and spermidine), or lipids may be linked to the oligonucleotides.

The randomized collection of oligonucleotides can be prepared by chemical synthesis of nucleic acids, synthesis of nucleic acids using an automatic nucleic acid synthesizer, synthesis of nucleic acids using amplification such as PCR, a combination thereof, or the like. For preparation of RNA from DNA, an RNA library can also be constructed by *in vitro* transcription using a double-stranded DNA library as a template and RNA polymerase such as T7 RNA polymerase. The randomized collection of oligonucleotides can also be prepared by techniques as described in R. Stoltenburg et al., Biomolecular Engineering 24: 381-403, 2007, for example.

A collection of oligonucleotides and a target molecule can be bound to each other by bringing the collection of oligonucleotides into contact with the target molecule. For example, they can be bound by mixing the target molecule with an aqueous solution of the collection of oligonucleotides and then performing incubation. Such an aqueous solution can be buffered, if necessary. Conditions for incubation are not particularly limited, and the incubation is preferably performed conveniently under a temperature condition of room temperature or 37°C. Furthermore, the reaction time is not particularly limited and generally ranges from about 1 minute to 30 minutes. A target molecule may be immobilized to a solid phase or a carrier. In this case, an immobilized target molecule may be brought into contact with a collection of oligonucleotides simultaneously or successively.

A target molecule may be any of low-molecular-weight compounds, high-molecular-weight compounds, living organism-derived substances, microorganism-derived substances, virus-derived substances, inorganic chemical species, organic molecules, and complexes of inorganic ion and organic molecule, as described above, and their types are not limited. More specifically, examples of a target molecule include sugars, lipids, oligopeptides, proteins, amino acids, polyamines, nucleic acids, and viruses. Examples of functional species that can be bound by a target molecule include antigens, antibodies, ligands, receptors, interacting proteins, pharmaceuticals, antibiotics, and cofactors. Specific examples of a target molecule are described in R. Stoltenburg et al., Biomolecular Engineering 24: 381-403, 2007.

### 2.2. Step (b)

In this step, a subcollection of oligonucleotides that bind to a target molecule as described above is selected.

A subcollection of oligonucleotides bound to a target molecule in the step (a) above can be separated from oligonucleotides that have not bound to the target molecule, by using an immobilized target molecule as described above and by washing. That is, the free oligonucleotides unbound to the target molecule are removed by washing, while the subcollection of oligonucleotides bound to the immobilized target molecule can be selectively recovered by a simple solid-liquid separation means (e.g., filtration or centrifugation). Alternatively, unbound oligonucleotides may be digested using, as a means instead of washing, an enzyme cleaving DNA or RNA in a sequence-specific manner (e.g., RNaseA, RNaseT1, HaeIII, single-chain nucleic acid cleaving nuclease (e.g., Mung Bean Nuclease, S1 nuclease, or P1 nuclease), DNaseI, a restriction enzyme [e.g., MazF] for single-stranded RNA, or uracil-DNA glycosylase), so that free oligonucleotides are efficiently removed. In this manner, after removal of the oligonucleotides unbound to the target molecule, a subcollection of oligonucleotides (i.e., a group of nucleic acid aptamers) bound to the target molecule can be selected. This is specifically described in Examples 2 to 6 as described later.

In the above-described immobilization, a target molecule is attached to a solid phase or a carrier (or a support), which has an arbitrary shape or an appropriate size. Examples of the solid phase or carrier include, but are not limited to, metals (e.g., magnetic metals and ceramics), polymers or resins (e.g., latex, ion exchange resins, and photocrosslinkable polymers), polysaccharides (e.g., agarose and carrageenan), and minerals (e.g., silica gel and porous glass). Examples of immobilization methods include a method of covalent bonding, a method of non-covalent bonding, and a method of entrapment.

In the method of covalent bonding, a functional group that is capable of binding to a functional group of a target molecule should be present on the surface of a solid phase or a carrier. Examples of a functional group include, but are not limited to, amino, formyl, carboxyl, hydroxy, thiol, and N-hydroxy succinimidyl.

Examples of non-covalent bonding include ionic bonding, hydrophobic bonding, and physical bonding. In ionic bonding, an electrical attraction between positive charge and negative charge occurs. Hydrophobic bonding or physical bonding is such a bonding that, in the case of a porous carrier, a substance is adsorbed to the inner surfaces of pores.

The entrapment method involves entrapping a substance within a gel having fine spaces, for example.

As a means for eluting the above-selected subcollection of oligonucleotides from the oligonucleotides bound to the immobilized target molecule, a means of using a surfactant, a means of increasing ion intensity, or a means of increasing a temperature can be used, for example.

Meanwhile, examples of a method for selecting oligonucleotides, in a case that no target molecule is immobilized, include a method that involves performing ultrafiltration on membranes having different molecular weight cutoffs, a method that involves, in a case that a target molecule is bound to a carrier, performing centrifugation to precipitate the carrier and then removing the supernatant containing unbound oligonucleotides, and a method that involves, in a case that magnetic beads and a target molecule are bound to each other, magnetically selecting and washing the carrier, the target molecule, and oligonucleotides bound thereto.

In the present invention, as a collection of oligonucleotides binding to a target molecule, a collection of oligonucleotides to which a modified nucleotide, peptide, amino acid, sugar, polyamine (e.g., spermin or spermidine), or lipid has been linked or a collection of oligonucleotides that are DNA/RNA chimeras can be selected. The selection of a collection of oligonucleotides can be performed only with the feature of the present invention such that the number of selection steps is one, and it cannot be achieved by conventional methods such as the SELEX method that requires multiple selections (i.e., repetition of selection). This is because the information on a collection of oligonucleotides that are DNA/RNA chimeras, modified nucleoetides or the like, or connected substances, are lost by the 2^{nd} and the following selection steps.

Oligonucleotides that are selected and eluted as described above can be amplified, if necessary. The method of the present invention does not always require amplification. This is because aptamer sequence information can be obtained without amplification in some cases. As a method for amplification, various gene amplification methods including PCR, LAMP, SMAP, and the like can be employed. In particular, the PCR method is described below.

PCR comprises repeating a series of steps of denaturation (chain melting) for generating a single-stranded template, primer annealing, and primer elongation, using thermostable DNA polymerase such as Thermus aquaticus (Taq) DNA polymerase. Typical 3-step PCR protocols comprise denaturation at 94-98°C for 5 seconds or more, primer annealing at 50-65°C for 10-60 seconds, and primer elongation at a temperature at which polymerase exhibits its high activity (72°C in the case of Taq DNA polymerase) for 15-120 seconds or more. For amplification, PCR is performed for about 20-40 cycles each consisting of the 3 above steps. In addition to a pair of primers and a nucleic acid to be amplified, the reaction mixture contains dNTPs (where "N" is A, T, C, or G), thermostable polymerase, divalent cation (Mg²⁺), and a buffering agent. When a subject is RNA, reverse transcriptase is also added. The volume of the reaction mixture generally ranges from 10 µl to 100 µl. Apparatuses (e.g., thermal cycler) for appropriate cycle conditions, reagent concentrations, primer design, and typical cyclic amplification reaction are commercially available. Hence, the use of such an apparatus is desired. Moreover, PCR technology is described in detail in Ausubel, F. Current Protocols in Molecular Biology (1988) Chapter 15: "The Polymerase Chain Reaction," J. Wiley (New York, U.S.A.), for example.

Primers to be used in the above PCR can be designed based on specific fixed sequences of X₁ and Xₙ in the above-described general formula. Specifically, upon PCR, primer sequences capable of annealing to such specific sequences can be used.

Moreover, when an oligonucleotide containing modified nucleotides is amplified by PCR, modified nucleoetides (e.g., methylated nucleotides) that can be amplified with an aptamer as a template can be used.

Also, amplification is desirably performed by a gene amplification method such as PCR, but sequence analysis may also be directly performed without performing amplification.

### 2.3. Step (c)

In this step, each sequence of the above-selected subcollection of oligonucleotides is examined. This step is an important step for characterizing the present invention.

A collection of oligonucleotides binding to a target molecule selected in step (b) above is amplified as necessary and then the nucleotide sequences of nucleic acids are examined and analyzed.

The term "examine (each sequence of a subcollection of oligonucleotides)" as used herein refers to determining nucleotide sequences of the oligonucleotides, or, through cleavage with an enzyme or the like, obtaining information on a part of the sequences. The nucleotide sequences of nucleic acids can be determined using known methods, commercially available sequencing devices, or the like. Alternatively, sequence information may also be examined by a method that involves measuring the mass spectrum of a nucleic acid, a method that involves cleaving a nucleic acid with a restriction enzyme, followed by electrophoresis, or the like. In any event, all methods by which the affinity spectrum of a target oligonucleotide can be obtained are preferably used in this step. The term "affinity spectrum" as used herein refers to a spectrum characterizing an oligonucleotide binding to a target molecule. Examples of such an affinity spectrum include a spectrum showing the relationship between the nucleotide sequences (which may be a randomized portion alone) of oligonucleotides, which are obtained by next-generation sequencing, and the number of oligonucleotides, for example.

A preferable method for determining nucleotide sequences is a method that uses a next-generation sequencer. Such a sequencer is commercially available, such as 454FLX (Roche Diagnostics), Genome Analyzer (Illumina, Inc.), or SOLiD (Applied Biosystems). These apparatuses can consecutively read the number of nucleotides, such as 400 nucleotides or 36 nucleotides (or the number of nucleotides greater than these examples, such as 1,000 or more nucleotides). The total number of nucleotides that can be read at one reading is one hundred million nucleotides, 1 to 2 billion nucleotides, or the like. Following amplification of DNA, or following conversion of RNA to cDNA, in the case of RNA, by reverse transcription/PCR (RT-PCR), an enormous number of sequences are determined using the next-generation sequencer. On a conceptual basis, Deep-Oligo-cap-sequencing, Whole-transcriptome, or the like is known, for example (Experimental Medicine (Jikken Igaku) Vol. 27, No.1, 2009, YODOSHA, Japan). Such a method using the next-generation sequencer can be said to be a particularly advantageous method for precise detection and selection of specific aptamers having high affinity, since the affinity spectra of a huge number of oligonucleotides for a target molecule can be obtained by the method.

### 2.4. Step (d)

In this step, sequence information characteristic to oligonucleotides having high affinity for a target molecule is extracted from the sequences of oligonucleotide selected and examined in the above-described steps.

Specifically, this step comprises extracting sequence information characteristic to oligonucleotides having high affinity for a target molecule by comparing the sequences of a subcollection of oligonucleotides binding to the target molecule, with the sequences of a collection of oligonucleotides obtained with a molecule other than the target or without the target.

To extract the characteristic information of oligonucleotides, a statistical processing for finding a common sequence of oligonucleotides bound to a target molecule, a technique that involves performing spectrum analysis based on affinity spectra, and the like can be employed, for example.

In Examples described later, roughly 10,000,000-20,000,000 oligonucleotide sequences obtained in step (b) were analyzed exhaustively using a next-generation sequencer. Specifically, a sequence corresponding to a randomized portion of a nucleic acid RNA which had been separately added to respective tubes, was organized with a barcode sequence indicating the corresponding tube, so that analysis was conducted. More specifically, the affinity spectra of a subcollection of oligonucleotides specifically binding to IgG (as a target molecule) and magnetic beads, and the affinity spectra of a subcollection of oligonucleotides binding to only magnetic beads were obtained. From the information of the affinity spectra, a set of nucleic acid sequences specifically binding to IgG was extracted.

### 2.5. Step (e)

In this step, a target molecule is identified based on the sequence information extracted from affinity spectra in step (d) above.

Based on sequences characteristic to a group of nucleic acid aptamers specifically binding to a target molecule, information of target molecules and nucleic acid aptamer sequences is converted into a database. Thus, a target molecule can be identified from the database only by obtaining an affinity spectrum, or, a nucleic acid aptamer specifically binding to a target molecule can also be identified from the database.

A target molecule may be any of low-molecular-weight compounds, high-molecular-weight compounds, living organism-derived substances, microorganism-derived substances, virus-derived substances, inorganic chemical species, organic molecules, and complexes of inorganic ion and organic molecule, as described above. Examples of a target molecule include sugars, lipids, oligopeptides, proteins, amino acids, and nucleic acids. More specific examples include antigens, antibodies, ligands, receptors, interacting proteins, pharmaceuticals, antibiotics, and cofactors.

Examples of nucleic acid aptamers binding to such a target molecule include nucleic acid aptamers specifically binding to a peptide, an amino acid, a sugar, a polyamine, or a lipid. These nucleic acid aptamers are also effective for, in addition to separation of a target molecule, introduction of a target molecule into a cell, and the like, recognition of a target molecule where it is a low-molecular-weight compound that is difficult to be recognized by an antibody, and recognition of a virus type within a short time where a target molecule is a virus.

### 3. Method for screening for nucleic acid aptamer

The present invention further provides a method for screening for a nucleic acid aptamer specifically binding to a target molecule from a collection of oligonucleotides with the use of the above method of the present invention.

Specifically, the method comprises identifying or specifying a target molecule by the method comprising the following steps (a) to (e) of:
(a) bringing a target molecule into contact with a collection of oligonucleotides comprising multiple nucleic acid aptamers that have different randomized sequences;
(b) selecting a subcollection of oligonucleotides binding to the above target molecule;
(c) examining each of sequences of the above-selected collection of oligonucleotides;
(d) extracting sequence information characteristic to the subcollection of oligonucleotides having affinity for the target molecule from the above-selected oligonucleotide sequences; and
(e) identifying the target molecule based on the above sequence information,
and then screening for and selecting a nucleic acid aptamer specifically binding to the target molecule from the above collection of oligonucleotides.

The above selection can be performed by labeling a target molecule with a label, such as a fluorescent substance or a radio isotope, and then detecting the signals, for example. Known labels can be used as such labels.

### 4. Method for quantifying target molecule

The present invention further provides a method for quantifying a target molecule, comprising adding a sample containing a target molecule to a nucleic acid aptamer not binding to the target molecule and a nucleic acid aptamer specifically binding to the target molecule, treating both nucleic acid aptamers together with nuclease, and then examining the ratio of the amounts of both nucleic acid aptamers in order to quantify the target molecule.

In one embodiment, the present invention provides a method for quantifying a target molecule, wherein a target molecule is quantified using a nucleic acid aptamer specifically binding to the target molecule, which is obtained by the above method for screening for a nucleic acid aptamer.

Specifically, as described later in Example 5, one or more aptamers specifically binding to a target molecule are bound to the target molecule, while an aptamer not binding to the target molecule is used as a comparative control, both of the aptamers are treated with nuclease, and then the ratio of the amounts of both nucleic acid aptamers after treatment is examined, so that the target molecule can be quantified. Nuclease used herein is a nuclease as described above.

The use of multiple aptamers which are different in specificity and are obtained by the method of the present invention, enables specific quantification with high accuracy. With the steps of the method for quantification, a nucleic acid aptamer is bound to a target molecule and then the resulting aptamer/target molecule complex is digested with nuclease. Because the aptamer having affinity for the target molecule is not easily digested, whereas the control aptamer not binding to the target molecule is digested, the amount of the target molecule can be found by observing the ratio of the amount of the former aptamer to that of the control aptamer. In Examples, digested nucleic acid aptamers were amplified by PCR, but this amplification may not always be performed.

The ratio of the amounts of the aptamers may be found by cleaving with a restriction enzyme and then performing gel electrophoresis as described in Examples. Alternatively, the ratio may also be found by MASS spectral analysis, sequencing analysis, or the like.

A possible application of the quantification method involves using different aptamers for multiple different target molecules and thus examining multiple target molecules simultaneously. Furthermore, the use of multiple different nucleic acid aptamers having affinity for a single target molecule enables highly specific and highly accurate quantification of the target molecule.

### Example

The present invention will be described in detail with reference to working examples, but it should not be construed that the scope of the present invention is limited to the working examples.

### Example 1

### Screening of aptamer specifically binding to IgG

The following RNAs were used as nucleic acid libraries.

5'-CGAUAGGUGUUGAGN₁N₂N₃N₄N₅N₆N₇CUCAGCGCUUGUUG-3'(SEQ ID NO: 1)
5'-CGAUAGGUGUUGAGN₁N₂N₃N₄N₅N₆CUCAGCGCUUGUUG-3'(SEQ ID NO: 2)
5'-CGAUAGGUGUUGAGN₁N₂CGCUUCGGCGN₃N₄N₅N₆N₇CUCAGCGCUUGUUG-3' (SEQ ID NO: 3)
(where "N" denotes a ribonucleotide sequence comprising a mixture of G, A, U, and C.)
Eight (8) tubes were prepared (where tubes 1 to 4: for negative control, tubes 5 to 8: for binding to IgG). Fifty (50) µl of DynaBeads Protein G (Invitrogen Corporation) was added to each tube, 1 ml of binding buffer was added, and then the solution was stirred at 37°C for 1 hour, followed by blocking treatment. Subsequently, 5 µl of mouse IgG (5 mg/ml) was added to each of tubes 5 to 8, followed by stirring at 37°C for 1 hour. After washing twice with 1 ml of binding buffer, 1 ml of binding buffer pre-incubated at 37°C, and 8 µl of the nucleic acid library (100 µM) (SEQ ID NO: 1) or 1.25 µl of the nucleic acid library (100 µM) (SEQ ID NO: 2), or 8 µl of the nucleic acid library (100 µM) (SEQ ID NO: 3) that had been subjected to 5-minutes denaturation and then 2-minutes cooling with ice were added to each tube. After stirring at 37°C for 30 minutes, washing was performed 5 times using 1 ml of washing buffer under washing conditions of 37°C and 30 minutes. After washing once with sterilized water, 50 µl of sterilized water was added and then incubation was performed at 90°C for 3 minutes, so that RNA that had bound was eluted.

In each of tubes 1 to 8, a reverse transcription reaction was performed using the eluted RNA as a template and a DNA primer (5'-CAAGCAGAAGACGGCATACGAGCTCTTCCGATCTCAACAAGCGCTGAG-3' (SEQ ID NO: 4)) in a reaction volume of 20 µl. ReverTra Ace^{®} (Toyobo Co., Ltd.) was used as reverse transcriptase. For control, reverse transcription was performed in each of 4 independent tubes (tubes 9 to 12) similarly using RNA diluted to an amount of 1/10⁶ of the RNA used for selection.
PCR was performed using 1 µl of cDNA, the DNA primer (SEQ ID NO: 4), and the DNA primer (any one of SEQ ID NOS: 5 to 16).

The primer combinations used for the tubes are as follows.

Tube 1: SEQ ID NO: 4, SEQ ID NO: 5 [5'-ACACTCTTTCCCTACACGACGCTCTTCCGATCT aaa CGATAGGTGTTGAG-3']
Tube 2: SEQ ID NO: 4, SEQ ID NO: 6 [5'-ACACTCTTTCCCTACACGACGCTCTTCCGATCT aat CGATAGGTGTTGAG-3']
Tube 3: SEQ ID NO: 4, SEQ ID NO: 7 [5'-ACACTCTTTCCCTACACGACGCTCTTCCGATCT aag CGATAGGTGTTGAG-3']
Tube 4: SEQ ID NO: 4, SEQ ID NO: 8 [5'-ACACTCTTTCCCTACACGACGCTCTTCCGATCT aac CGATAGGTGTTGAG-3']
Tube 5: SEQ ID NO: 4, SEQ ID NO: 9 [5'-ACACTCTTTCCCTACACGACGCTCTTCCGATCT ata CGATAGGTGTTGAG-3']
Tube 6: SEQ ID NO: 4, SEQ ID NO: 10 [5'-ACACTCTTTCCCTACACGACGCTCTTCCGATCT att CGATAGGTGTTGAG-3']
Tube 7: SEQ ID NO: 4, SEQ ID NO: 11 [5'-ACACTCTTTCCCTACACGACGCTCTTCCGATCT atg CGATAGGTGTTGAG-3']
Tube 8: SEQ ID NO: 4, SEQ ID NO: 12 [5'-ACACTCTTTCCCTACACGACGCTCTTCCGATCT atc CGATAGGTGTTGAG-3']
Tube 9: SEQ ID NO: 4, SEQ ID NO: 13 [5'-ACACTCTTTCCCTACACGACGCTCTTCCGATCT aga CGATAGGTGTTGAG-3']
Tube 10: SEQ ID NO: 4, SEQ ID NO: 14 [5'-ACACTCTTTCCCTACACGACGCTCTTCCGATCT agt CGATAGGTGTTGAG-3']
Tube 11: SEQ ID NO: 4, SEQ ID NO: 15 [5'-ACACTCTTTCCCTACACGACGCTCTTCCGATCT agg CGATAGGTGTTGAG-3']
Tube 12: SEQ ID NO: 4, SEQ ID NO: 16 [5'-ACACTCTTTCCCTACACGACGCTCTTCCGATCT age CGATAGGTGTTGAG-3']
   Sequences denoted with lowercase letters correspond to bar code sequences for distinguishing each of PCR products in the following sequencing.

PCR was performed using a thermal circular (Biometra), after keeping the temperature at 95°C for 1 minute, for 35 cycles each consisting of 95°C for 15 seconds, 55°C for 30 seconds, and 72°C for 30 seconds. As a PCR reagent, THUNDERBIRD qPCR Mix (Toyobo Co., Ltd.) was used.

The PCR product in each tube was confirmed by agarose gel electrophoresis. The PCR products were mixed together to produce a mixture containing roughly equal amounts of each PCR product. Ten (10) ng of the PCR product as a template was further amplified by 10 cycles of PCR. As a set of primers, 5'-AATGATACGGCGACCACCGAGATCTACACTCTTTCCCTACACGACGCTCTTCCGA TCT-3' (SEQ ID NO: 17) and 5'-CAAGCAGAAGACGGCATACGAGCTCTTCCGATCT-3' (SEQ ID NO: 18) were used.

The thus amplified PCR product was subjected to sequence analysis using an Illumina Genome Analyzer (Illumina, Inc.) with the DNA primer, 5'-ACACTCTTTCCCTACACGACGCTCTTCCGATCT-3' (SEQ ID NO: 19).

The sequences of the thus-obtained about 20,000,000 clusters (i.e., about 20,000,000 oligonucleotides) were analyzed. Analysis was conducted based on the sequences corresponding to randomized regions of the nucleic acid RNAs added to tubes and the barcode sequences indicating the tubes corresponding thereto. For each of tubes 1 to 4 (DynaBeads alone), tubes 5 to 8 (DynaBeads + IgG), and tubes 9 to 12 (sequences before selection), the average number of clusters corresponding to each sequence (i.e., the average number of detected oligonucleotides), and p-value were calculated. Nucleic acid sequences specifically binding to IgG (i.e., specific nucleic acid sequences in tubes 5 to 8) were extracted. The results are shown in Table 1.

**Table 1**

| | Beads alone | IgG + Beads | Before selection | | |
|---|---|---|---|---|---|
| Nucleotide sequence of randomized portion | Average number of clusters in tubes (1-4) | Average number of clusters in tubes (5-8) | Average number of clusters in tubes (9-12) | p-value (Beads vs IgG+Beads) | p-value (IgG+Beads vs Before selection) |
| GCCTGT | 688 | 1502 | 20 | 0.309 | 0.001 |
| TTGGAT | 610 | 962 | 21 | 0.589 | 0.004 |
| GCACCG | 407 | 249 | 26 | 0.721 | 0.005 |
| TGGGTT | 227 | 817 | 18 | 0.063 | 0.009 |
| GCTGTG | 676 | 297 | 17 | 0.608 | 0.009 |
| GTGGAT | 24 | 1563 | 105 | 0.014 | 0.013 |
| TTCGAT | 44 | 548 | 42 | 0.019 | 0.016 |
| GTGGGT | 136 | 730 | 48 | 0.024 | 0.021 |
| GGTGAT | 392 | 740 | 7 | 0.446 | 0.022 |
| GGCGAT | 155 | 691 | 36 | 0.037 | 0.022 |
| GGGTAT | 37 | 787 | 38 | 0.039 | 0.038 |
| GTTGGT | 25 | 605 | 65 | 0.035 | 0.04 |
| CCTGTT | 257 | 799 | 16 | 0.143 | 0.045 |
| TTGGTT | 23 | 713 | 25 | 0.048 | 0.048 |
| TGGCAT | 375 | 563 | 82 | 0.65 | 0.05 |
| CCTGTC | 1105 | 303 | 7 | 0.517 | 0.061 |
| GTTGGG | 15 | 405 | 19 | 0.071 | 0.073 |
| TGGGCT | 78 | 605 | 201 | 0.014 | 0.123 |
| GGTGCG | 376 | 125 | 12 | 0.525 | 0.24 |
| TTGGGA | 47 | 111 | 97 | 0.406 | 0.895 |

As seen in Table 1, in many cases when tubes containing IgG + DynaBeads are compared with tubes containing DynaBeads alone, the average number of clusters is (IgG + DynaBeads) > (DynaBeads alone) and p-values are 0.05 or less. These affinity spectra were statistically significant in relative to the affinity spectra in the case of beads alone or the affinity spectra before selection, indicating that the presence of IgG was detected. Furthermore, it is understood that, with the use of this method, an aptamer specific to an arbitrary target molecule can be conveniently screened, or, a target molecule having high affinity for or specifically binding to an aptamer can be detected.

In Table 1, the sequences of randomized regions indicate those read using a next-generation sequencer. In actual aptamer sequences, T is U.

### Example 2

### Selection of target molecule using nucleic acid library and sequence analysis of bound nucleic acid

As a nucleic acid library containing modified nucleic acids, the following nucleic acids were used.

5'-cgcctctccgagagtgtNNNNNNNacaUtctUggagaggUg-3' (SEQ ID NO: 21)
5'-cgcctctccgagagtgtnnnnnnacactctcggagaggcg-3' (SEQ ID NO: 22)
5'-cgcctctccgagagtgtnnnnnnnacactctcggagaggcg-3' (SEQ ID NO: 23)
(In the 3 sequences, uppercase letters indicate RNA and lowercase letters indicate DNA. "N" denotes a ribonucleotide sequence comprising a mixture of G, A, U, and C, and "n" indicates a nucleotide sequence comprising a mixture of g, a, t, and c.)

### <Preparation of nucleic acid library and binding to substance>

Each nucleic acid library (2 µl(100 µM)) was added to 200 µlof binding buffer (10 mM Tris-HCl (pH 7.5), 150 mM NaCl, 10 mM MgCl₂, 0.05% Tween 20). After 2-minutes denaturation at 98°C, the mixture was left to stand at 4°C for 1 minute, and then 10 mg/ml ssDNA (as a blocking agent) was added. Subsequently, mouse IgG-conjugated protein G beads, protein G beads, streptavidin beads, a terminally biotinylated Flag peptide (DYKDDDDKGG-Biotin, where DYKDDDDKGG is SEQ ID NO: 39)-conjugated streptavidin beads, or control beads were added. The samples were shaken at room temperature for 30 minutes so that aptamers were bound. Washing was then performed 4 times with a binding buffer.

Beads used were DynaBeads (Invitrogen Corporation).

### <Elution procedure>

After washing once with 200 µl of distilled water, the nucleic acid library bound to protein G beads or control beads was eluted by suspending in 15 µl of distilled water and then heating at 98°C for 2 minutes.

The nucleic acid library that had bound to mouse IgG, the Flag peptide, or streptavidin was eluted by adding 50 µl of binding buffer and 5 µl of mouse IgG (2.5 mg/ml), the Flag peptide (Sigma) (10 mM), or streptavidin (Roche) (2.5 mg/ml), and then shaking the mixture for 30 minutes. After elution, phenol chloroform extraction and ethanol precipitation were performed, and then the precipitate was dissolved in 15 µl of distilled water.

### <Reverse transcription reaction>

The following reverse transcription reaction was further performed for the nucleic acid aptamer (SEQ ID NO: 21) in which the randomized region was RNA.

Reverse transcription and enzyme inactivation were performed using 5'-CAAGCAGAAGACGGCATACGAGCTCTTCCGATCTCACCTCTCCAAGAATGT-3' (SEQ ID NO: 24) (final concentration: 1 µM), as a reverse transcription primer, and a PrimeScript II 1^{st} strand cDNA Synthesis Kit (Takara Bio Inc., Japan) under conditions of 42°C for 60 minutes, 52°C for 30 minutes, and 98°C for 5 minutes. The sample was then subjected to ethanol precipitation and then dissolved in 10 µl of distilled water.

### <PCR amplification procedure>

The thus eluted nucleic acid aptamer was amplified using the following primer set.

5'-AATGATACGGCGACCACCGAGATCTACACTCTTTCCCTACACGACGCTCTTCCGA TCT [Barcode sequence] CGCCTCTCCGAGAGTGT-3' (SEQ ID NO: 25 to SEQ ID NO: 28)
5'-CAAGCAGAAGACGGCATACGAGCTCTTCCGATCTCACCTCTCCAAGA ATGT-3' (SEQ ID NO: 24)
(where [Barcode sequence] is an arbitrary sequence consisting of 4-7 nucleotides used for distinguishing samples upon analysis with a next-generation sequencer. Primers used have barcode sequences that vary from one sample to another.)
PCR was performed using a thermal circular (Biometra), after keeping the temperature at 96°C for 1 minute, for 1 cycle of 95°C for 15 seconds, 46°C for 20 seconds, and 72°C for 60 seconds, then 35 cycles each consisting of 95°C for 15 seconds and 72°C for 60 seconds. The PCR reagent used was KOD-Neo-plus (Toyobo Co., Ltd., Japan).

The amplified PCR product was subjected to sequence analysis using an Illumina Genome Analyzer (Illumina, Inc.) and the DNA primer 5'-ACACTCTTTCCCTACACGACGCTCTTCCGATCT-3' (SEQ ID NO: 29).

The sequences of the obtained about 10,000,000-to-20,000,000 clusters (i.e., about 10,000,000-to-20,000,000 oligonucleotides) were analyzed. The sequences of the nucleic acid library that had bound to mouse IgG, Flag peptide, or streptavidin were analyzed based on sequences corresponding to the randomized regions of the nucleic acid library added to tubes and barcode sequences indicating the tubes corresponding thereto. The nucleic acid sequences specifically binding to each substance and the average number of clusters corresponding to each of the nucleic acid sequences (the average number of each oligonucleotide detected) are shown in Table 2-1 and Table 2-2.

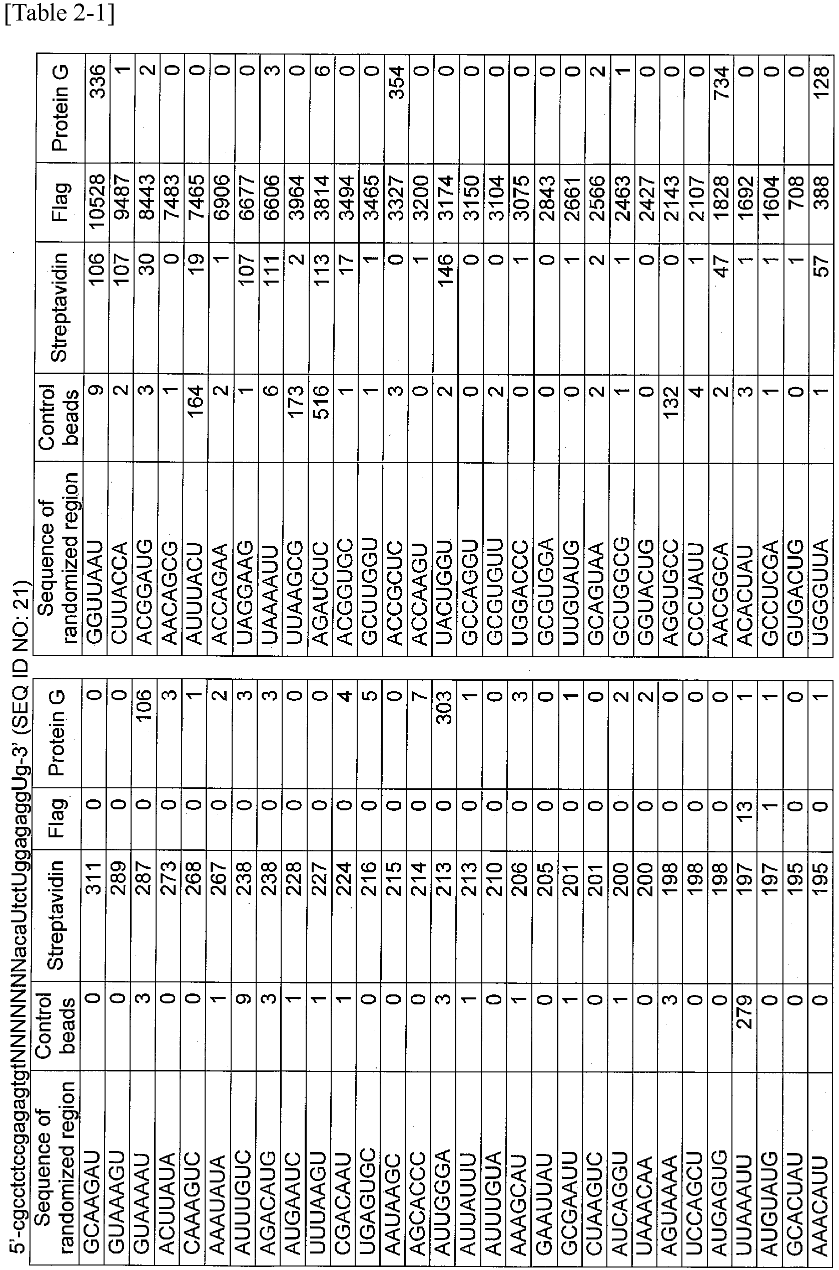

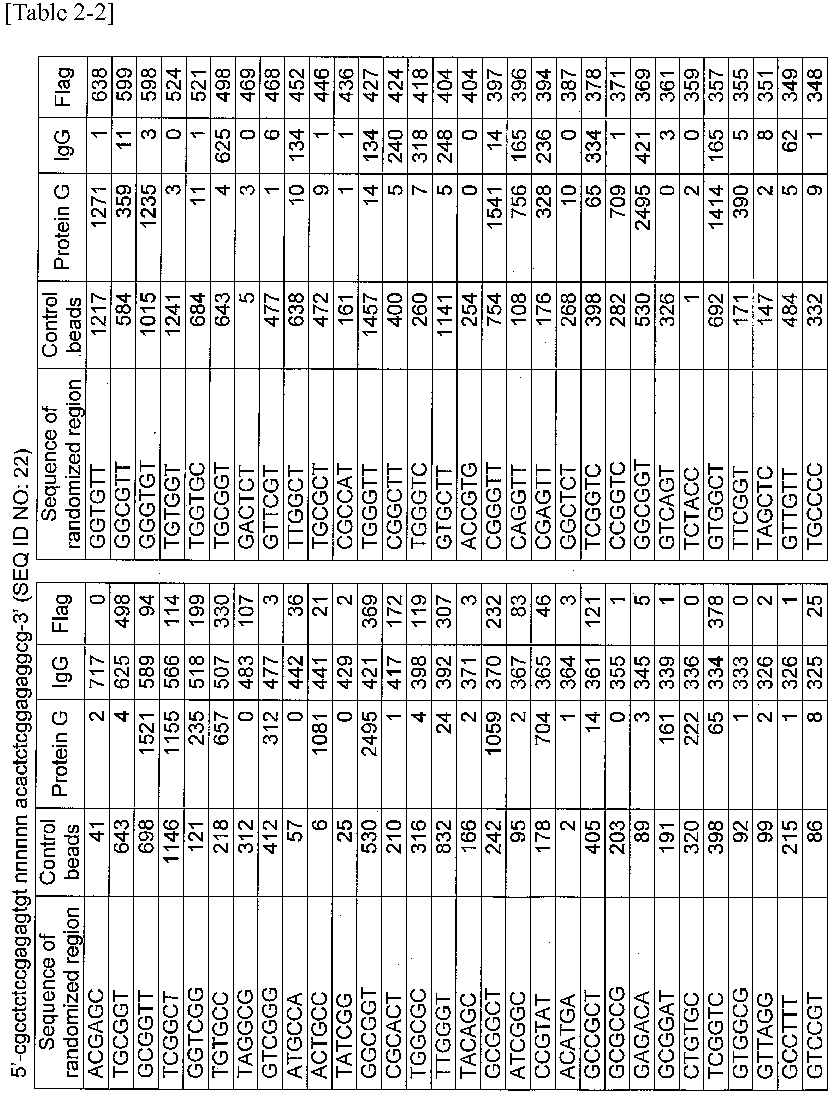

Table 2-1 shows the top 30 sequences of and the number of sequences of randomized regions of the nucleic acid aptamer (SEQ ID NO: 21) bound to streptavidin beads (left), and, Flag peptide beads (right).

Table 2-2 shows the top 30 sequences of and the number of sequences of randomized regions of the nucleic acid aptamer (SEQ ID NO: 22) bound to IgG beads (left), and, Flag peptide beads (right).

Patterns characteristic of each target were obtained.

### Example 3

### Discrimination between mouse IgG and rat IgG using DNA nucleic acid library

As a nucleic acid library having modified nucleic acids, the following nucleic acid was used.

5'-cgcctctccgagagtgtNNNNNNNacaUtctUggagaggUg-3' (SEQ ID NO: 21)
(In this sequence, uppercase letters indicate RNA and lowercase letters indicate DNA. "N" denotes a ribonucleotide sequence comprising a mixture of G, A, U, and C)

### <Preparation of nucleic acid library and binding to substance>

Each nucleic acid library (2 µl (100 µM)) was added to 200 µl of binding buffer (10 mM Tris-HCl (pH 7.5), 150 mM NaCl, 10 mM MgCl₂, 0.05% Tween 20). After 2-minutes denaturation at 98°C, the mixture was left to stand at 4°C for 1 minute, and then 10 mg/ml ssDNA (blocking agent) was added. Subsequently, mouse IgG-conjugated protein G beads, or, rat-IgG-conjugated protein G beads, or, protein G beads were added. The mixtures were shaken at room temperature for 30 minutes so that aptamers were bound. Washing was then performed 4 times with a binding buffer.

### <Elution procedure>

After washing once with 200 µl of distilled water, the nucleic acid library that had bound to protein G beads was eluted by suspending in 15 µl of distilled water, and then heating at 98°C for 2 minutes.

The nucleic acid library that had bound to mouse IgG, Flag peptide, or, streptavidin was eluted by adding 50 µl of binding buffer and 5 µl of mouse IgG (2.5 mg/ml), rat IgG (2.5 mg/ml) or streptavidin (Roche) (2.5 mg/ml) and then shaking for 30 minutes. After elution, phenol chloroform extraction and ethanol precipitation were performed, and then the precipitate was dissolved in 15 µl of distilled water.

### <Reverse transcription reaction>

Reverse transcription and enzyme inactivation were performed using 5'-CAAGCAGAAGACGGCATACGAGCTCTTCCGATCTCACCTCTCCAAGAATGT-3' (SEQ ID NO: 24) (final concentration: 1 µM), as a reverse transcription primer, and a PrimeScript II 1^{st} strand cDNA Synthesis Kit under conditions of 42°C for 60 minutes, 52°C for 30 minutes, and 98°C for 5 minutes. The sample was then subjected to ethanol precipitation and then dissolved in 10 µl of distilled water.

### <PCR amplification procedure>

The thus eluted nucleic acid aptamer was amplified using the following primer set.

5'-AATGATACGGCGACCACCGAGATCTACACTCTTTCCCTACACGACGCTCTTCCGA TCT [Barcode sequence] CGCCTCTCCGAGAGTGT-3' (SEQ ID NO: 25 to SEQ ID NO: 28)
5'-CAAGCAGAAGACGGCATACGAGCTCTTCCGATCTCACCTCTCCAAGAATGT-3' (SEQ ID NO: 24)
(where [Barcode sequence] is an arbitrary sequence consisting of 4-7 nucleotides used for distinguishing samples upon analysis with a next-generation sequencer. Primers used have barcode sequences that vary from one sample to another.)
PCR was performed using a thermal circular (Biometra), after keeping the temperature at 96°C for 1 minute, for 1 cycle of 95°C for 15 seconds, 46°C for 20 seconds, and 72°C for 60 seconds, then 35 cycles each consisting of 95°C for 15 seconds and 72°C for 60 seconds. The PCR reagent used was KOD-Neo-plus (Toyobo Co., Ltd., Japan).

The amplified PCR product was subjected to sequence analysis using an Illumina Genome Analyzer (Illumina, Inc.) and the DNA primer 5'-ACACTCTTTCCCTACACGACGCTCTTCCGATCT-3' (SEQ ID NO: 29).

The sequences of the obtained about 10,000,000-to-20,000,000 clusters (i.e., about 10,000,000-to-20,000,000 oligonucleotides) were analyzed. The sequences of the nucleic acid library that had bound to mouse IgG, or, rat IgG were analyzed based on sequences corresponding to the randomized regions of the nucleic acid library added to tubes and barcode sequences indicating the tubes corresponding thereto. The nucleic acid sequences specifically binding to each substance, as well as the average number of clusters corresponding to each of the nucleic acid sequences (the average number of each oligonucleotide detected), are shown in Table 3.

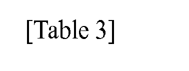

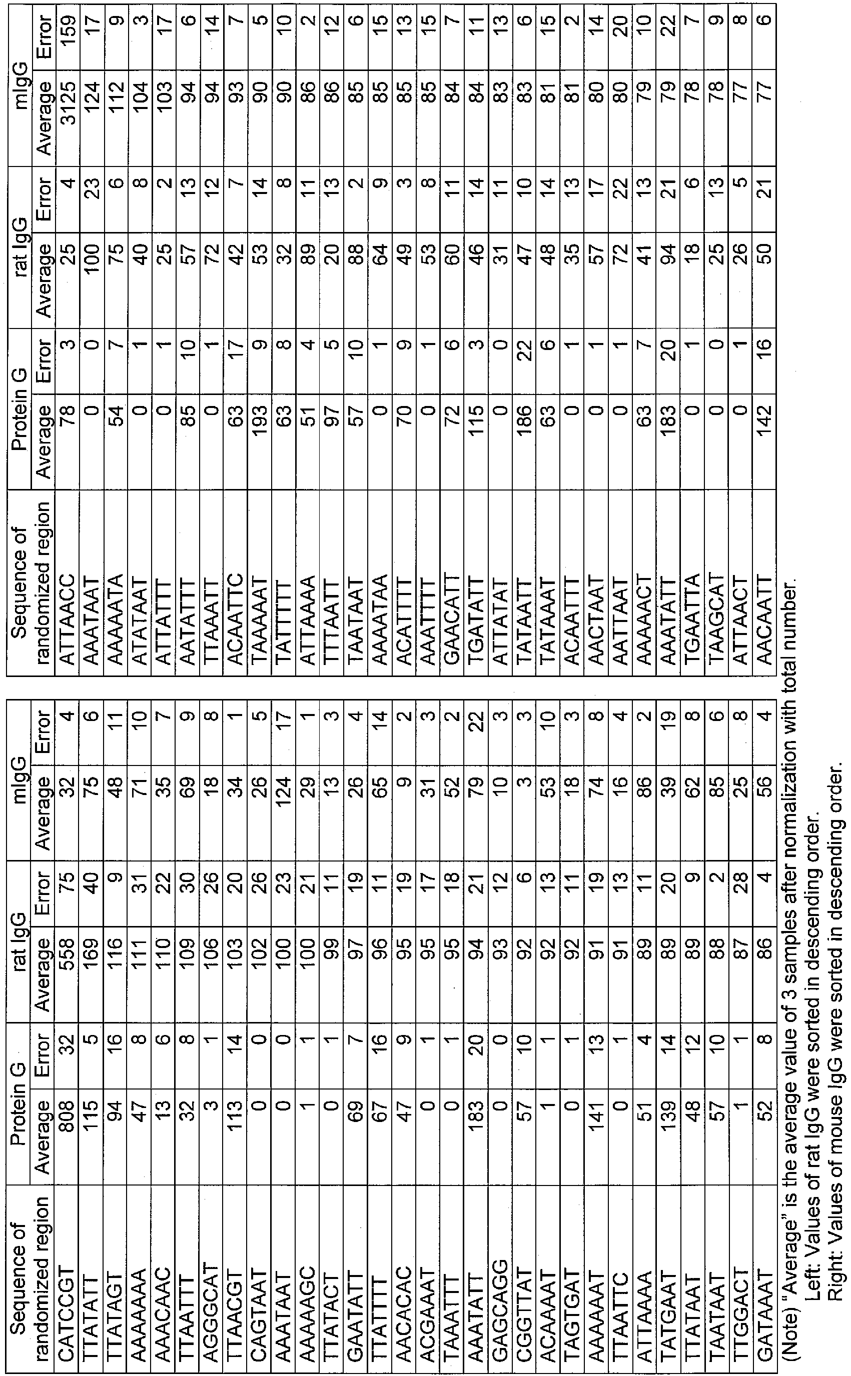

Table 3 shows the top 30 sequences of and the number of sequences of randomized regions of the nucleic acid aptamer bound to rat IgG beads (left), and, mouse IgG beads (right).

Characteristic patterns were exhibited differently even in the cases of closely related proteins such as rat IgG and mouse IgG.

### Example 4

Selection and sequence analysis using nucleic acid library containing modified nucleic acid
As a nucleic acid library containing modified nucleic acids, the following DNA nucleic acids were used.

5'-CGCCTCTCCGAGAGTGTCGCNNN[aminoC]NNNGCGACATTCTTGGAGAGGTG-3' (SEQ ID NO: 30)
5'-CGCCTCTCCGAGAGTGTCGCNNN[meC]NNNGCGACATTCTTGGAGAGGTG-3' (SEQ ID NO: 31)
(where "N" denotes a nucleotide sequence comprising a mixture of G, A, T, and C; [aminoC] denotes Amino modifier C6 dC (5'-Dimethoxytrityl-N-dimethylformamidine-5-[N-(trifluoroacetylaminohexyl)-3-acrylimido]-2'-deoxyCytidine,3'-[(2-cyanoethyl)-(N,N-diisopropyl)]-phosphoramidite) (Glen Research Corporation, U.S.A.); and [meC] denotes 5-methyl-dC (5-Methyl deoxyCytidine).)

### <Preparation of nucleic acid library and binding to IgG or protein G>

Each nucleic acid library (2 µl(100 µM)) was added to 200 µl of binding buffer (10 mM Tris-HCl (pH 7.5), 150 mM NaCl, 10 mM MgCl₂, 0.05% Tween 20). After 2-minutes denaturation at 98°C, the mixture was left to stand at 4°C for 1 minute, and then 10 mg/ml ssDNA (blocking agent) was added. Subsequently, mouse IgG-conjugated protein G beads, or, protein G beads (before conjugation of IgG) were added. The mixtures were shaken at room temperature for 30 minutes so that aptamers were bound. Washing was then performed 4 times with a binding buffer.

### <Elution procedure>

After washing once with 200 µl of distilled water, the nucleic acid library that had bound to protein G was eluted by suspending in 15 µl of distilled water, and then heating at 98°C for 2 minutes.

The nucleic acid library that had bound to mouse IgG was eluted by adding 50 µl of binding buffer and 5 µl of mouse IgG (0.5 ml/ml) and then shaking for 30 minutes. Phenol chloroform extraction and ethanol precipitation were then performed, and then the precipitate was dissolved in 15 µl of distilled water.

### <PCR amplification procedure>

The thus eluted nucleic acid aptamer was amplified using the following primer set.

5'-AATGATACGGCGACCACCGAGATCTACACTCTTTCCCTACACGACGCTCTTCCGA TCT [Bar code sequence] CGCCTCTCCGAGA GTGT-3' (SEQ ID NO: 25 to SEQ ID NO: 28)
5'-CAAGCAGAAGACGGCATACGAGCTCTTCCGATCTCACCTCTCCAAGAATGT-3' (SEQ ID NO: 24)
(where [Barcode sequence] is an arbitrary sequence of 4-7 nucleotides for distinguishing samples upon analysis using a next-generation sequencer. Primers used have barcode sequences that vary from one sample to another.)
PCR was performed using a thermal circular (Biometra), after keeping the temperature at 96°C for 1 minute, for 1 cycle of 95°C for 15 seconds, 46°C for 20 seconds, and 72°C for 60 seconds, then 35 cycles each consisting of 95°C for 15 seconds and 72°C for 60 seconds. The PCR reagent used was KOD-Neo-plus (Toyobo Co., Ltd., Japan).

The amplified PCR product was subjected to sequence analysis using an Illumina Genome Analyzer (Illumina, Inc.) and the DNA primer, 5'-ACACTCTTTCCCTACACGACGCTCTTCCGATCT-3' (SEQ ID NO: 29).

The sequences of the obtained about 10,000,000-to-20,000,000 clusters (i.e., about 10,000,000-to-20,000,000 oligonucleotides) were analyzed. The sequences of the nucleic acid library that had bound to IgG and protein G were analyzed based on sequences corresponding to the randomized regions of the nucleic acid library added to tubes and barcode sequences indicating the tubes corresponding thereto. Table 4 shows the nucleic acid sequences of the nucleic acid library specifically binding to IgG or protein G and the average number of clusters corresponding to each of the nucleic acid sequences (the average number of each oligonucleotide detected).

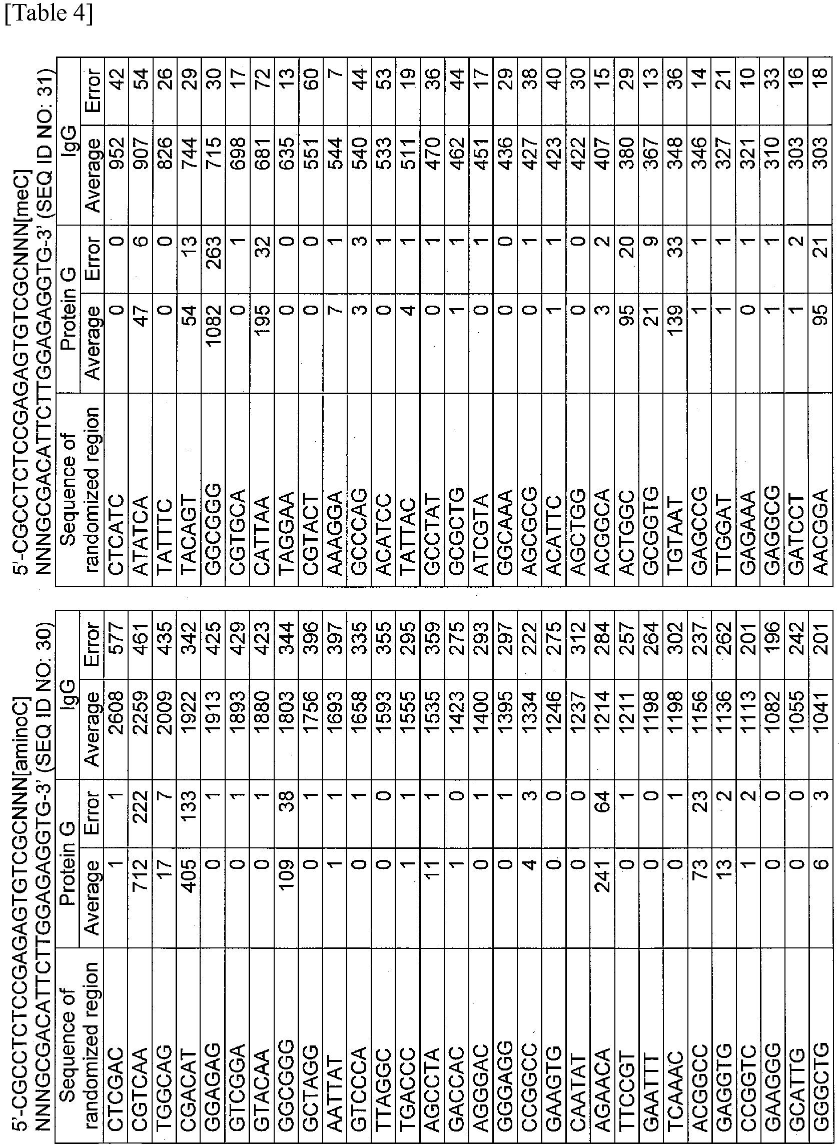

Table 4 shows the top 30 sequences of and the number of sequences of randomized regions of the nucleic acid aptamer bound to mouse IgG beads, containing two different modifications. Patterns differing depending on nucleic acid modification were obtained.

It was suggested that modification of nucleic acids can enhance sensitivity for detecting substances.

### [Example 5]

### Quantification of streptavidin using S1 nuclease and streptavidin aptamer

The following DNA nucleic acid was used as a streptavidin aptamer binding to streptavidin.

5'-TCTGTGAGACGACGCACCGGTCGCAGGTTTTGTCTCACAG-3' (SEQ ID NO: 32)
   The following DNA nucleic acid was used as a control aptamer not binding to streptavidin.

5'-TCTGTGAGACGACGCACCGACCGCAGGTTTTGTCTCACAG-3' (SEQ ID NO: 33)
The following solution was prepared and left to stand at room temperature for 30 minutes in order to bind the aptamer to streptavidin at different concentrations.

1 µl: 0.5% tween-20
1 µl: 1 nM streptavidin aptamer
1 µl: 1 nM control avidin aptamer
2 µl: 5 x S1 nuclease buffer
2 µl: streptavidin (0.5 mg/ml, 0.025 mg/ml, 0.0125 mg/ml, 0.0625 mg/ml, or none, in each tube)
2 µl: distilled water
Total: 9 µl
   (where 5 x S1 nuclease buffer: 250 mM NaCl, 100 mM MgCl₂, 25% glycerol, 250 mM Tris-HCl pH 7.5)
Subsequently, 1 µl of S1 nuclease (22.5 units/µl) (Takara, Japan) was added to each tube followed by 20-minutes reaction at 37°C, and then the reaction was stopped by heating at 98°C for 3 minutes.

S1 nuclease is an enzyme for cleavage of single-stranded nucleic acids. In the case of a streptavidin aptamer to which streptavidin has bound, cleavage by S1 nuclease is inhibited and thus digestion thereof is difficult. However, a control aptamer to which no streptavidin has bound is easily digested.

Samples after reaction were amplified by PCR using the following primer set.

5’-AATGATACGGCGACCACCGAGATCTACACTCTTTCCCTACACGACGCTCTTCCGA
TCTAAAACGCCTCTCCGAGAGTGT-3’ (SEQ ID NO: 34)

5’-CAAGCAGAAGACGGCATACGAGCTCTTCCGATCTCGCCTCTCCGAGACCGCTGT
GT-3’ (SEQ ID NO: 35)

PCR was performed, using a thermal circular (Biometra), after keeping the temperature at 96°C for 1 minute, for 1 cycle of 95°C for 15 seconds, 46°C for 20 seconds, and 72°C for 60 seconds, then 40 cycles each consisting of 95°C for 15 seconds and 72°C for 60 seconds. As a PCR reagent, KOD-Neo-plus (Toyobo Co., Ltd., Japan) was used.

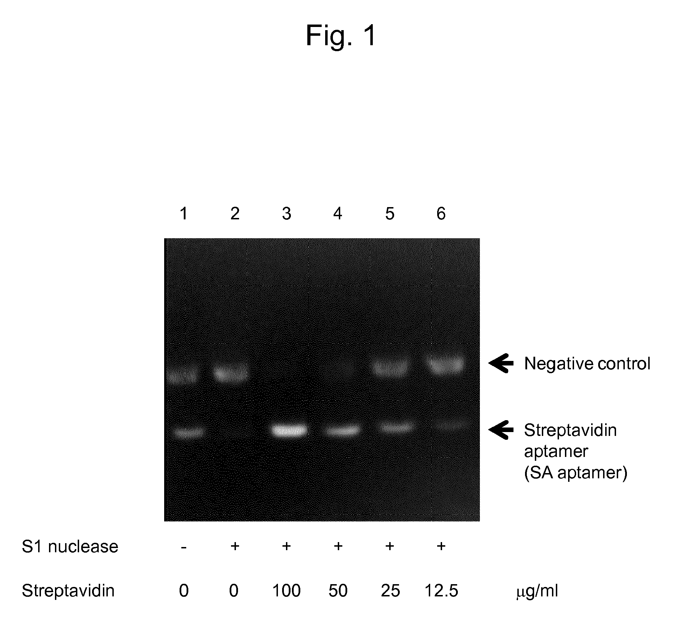

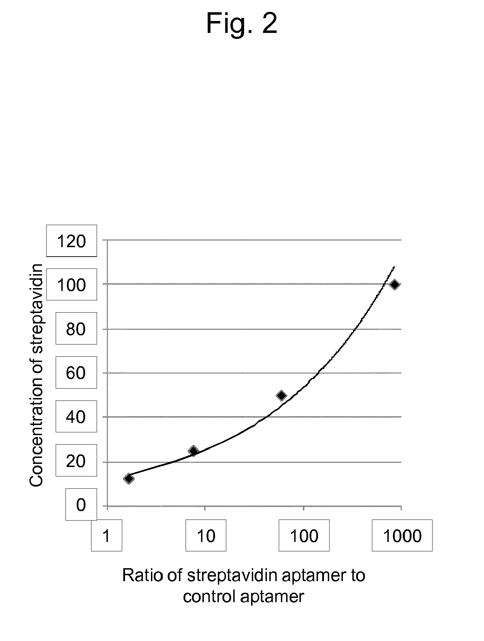

After PCR, products were digested with the *Msp* I restriction enzyme. The streptavidin aptamer had an *Msp* I site, but the control aptamer lacked the *Msp* I site. Samples after digestion were subjected to gel electrophoresis, so that the ratio of the amount of the streptavidin aptamer to the amount of the control aptamer could be determined. Fig. 1 shows the results of electrophoresis. Also, Fig. 2 shows a graph produced by reading and quantifying the density of bands and converting the obtained data into graph form. As shown in Fig. 2, the ratio of the amount of the streptavidin aptamer to the amount of the control aptamer varied depending on the concentration of streptavidin. It was revealed that the amount of streptavidin could be quantified using this technique.

### [Example 6]

### Obtaining sequence spectra for Flag peptide or biotin using S1 nuclease and randomized nucleic acid aptamer

The following DNA nucleic acid was used as a randomized nucleic acid aptamer.

5’-CGCCTCTCCGAGAGTGTCGCNNNNNNNNNNNNNNNNNGCGACATTCTTGGAGAG
GTG-3’ (SEQ ID NO: 36)

The following solution was prepared and left to stand at room temperature for 30 minutes in order to bind the aptamer to streptavidin at different concentrations.

1 µl: 0.5% tween-20
2 µl: 10 nM randomized nucleic acid aptamer
2 µl: 5 x S1 nuclease buffer
2 µl: Flag peptide (10 mM), or, biotin (10 mM)
2 µl: distilled water
Total: 9 µl
   (where 5 x S1 nuclease buffer: 250 mM NaCl, 100 mM MgCl₂, 25% glycerol, 250 mM Tris-HCl pH 7.5)
Subsequently, 1 µl of S1 nuclease (180 units/µl) (Takara, Japan) was added to each tube followed by 20-minutes reaction at 37°C, and then the reaction was stopped by heating at 98°C for 3 minutes.

S1 nuclease is an enzyme for cleavage of single-stranded nucleic acids. In the case of an aptamer to which a target has bound, cleavage by S1 nuclease is inhibited and thus digestion thereof is difficult. However, an aptamer to which no target has bound is easily digested.

Samples after reaction were amplified by PCR using the following primer set.

5’-AATGATACGGCGACCACCGAGATCTACACTCTTTCCCTACACGACGCTCTTCCGA
TCT [Barcode sequence] CGCCTCTCCGAGA GTGT-3’ (SEQ ID NO: 25 to SEQ ID NO:
28)

5'-CAAGCAGAAGACGGCATACGAGCTCTTCCGATCTCACCTCTCCAAGAATGT-3' (SEQ ID NO: 24)
(where [Barcode sequence] is an arbitrary sequence of 4-7 nucleotides for distinguishing samples upon analysis using a next-generation sequencer. Primers used have barcode sequences that vary from one sample to another.)
PCR was performed using a thermal circular (Biometra), after keeping the temperature at 96°C for 1 minute, for 1 cycle of 95°C for 15 seconds, 46°C for 20 seconds, and 72°C for 60 seconds, then 40 cycles each consisting of 95°C for 15 seconds and 72°C for 60 seconds. The PCR reagent used was KOD-Neo-plus (Toyobo Co., Ltd., Japan).

The amplified PCR product was subjected to sequence analysis using an Illumina Genome Analyzer (Illumina, Inc.) and the DNA primer 5'-ACACTCTTTCCCTACACGACGCTCTTCCGATCT-3' (SEQ ID NO: 29).

The sequences of the obtained about 10,000,000-to-20,000,000 clusters (i.e., about 10,000,000-to-20,000,000 oligonucleotides) were analyzed. The sequences of the nucleic acid library after digestion thereof in the presence of the Flag peptide, or, biotin were analyzed based on sequences corresponding to the randomized regions of the nucleic acid library added to tubes and barcode sequences indicating the tubes corresponding thereto. As shown in Table 5, the nucleic acid sequence pattern characteristic to each substance (Flag peptide or biotin) was observed.

**[Table 5]**

| No addition | | Flag | | Biotin | |
|---|---|---|---|---|---|
| Sequence of randomized region | Number of sequences | Sequence of randomized region | Number of sequences | Sequence of randomized region | Number of sequences |
| CCAATACTCATGCTC | 821 | ATGCAATAAACCACA | 1266 | CCTACCAATACCTTC | 917 |
| GTCCTAACCTCATTA | 804 | TGCTGTCTTTGAAAA | 1165 | CCTGCAGCTAACCTT | 849 |
| CTAATCTGGCAATAA | 799 | ACATTTCTTAGGGAC | 1123 | CTTATGCTCATGGAT | 846 |
| TTCCCCAAATTTGGC | 752 | TTCAGTGGTATACAA | 975 | TAAGCAGGAGAACCG | 845 |
| TGACCGCCTATATGC | 716 | GTATCAGACCCCCGA | 953 | AAACTCTCTCACTCA | 841 |
| GTCGGATCCACTCGA | 601 | TATAGTTCCAATGTG | 924 | ACTCCCCTTTTGCGC | 833 |
| TTGTGCTGACTGTCG | 571 | TCCTCCCGAGGGTGC | 920 | TTACCTTCATGCGGT | 814 |
| TTTGTCAATGTACGC | 518 | AGTGAAAAGAGATTG | 915 | TACCACCTTTTATAT | 812 |
| GGGTTATTGTTTGGG | 472 | CCGCGTGTCATCCCT | 828 | TTCACTCAAATAGCT | 798 |
| GATAACCCTAGCTCT | 469 | GTGGAGGCTGCATGC | 786 | GTACCACACCCTCTG | 795 |
| AGGAAATATGGCAAG | 449 | GGAGCGCGACCGGAG | 753 | TCATTAAAAGTACCC | 776 |
| TTACTTCATAGAGAA | 412 | CGAATCGTGCTTCAA | 727 | ATTTCCATTTCCTTT | 775 |
| TTTCCACCCCGACCA | 344 | CTATCGCTAGCCCGC | 687 | TTTTATCCCGGTTTT | 772 |
| GAAGGATTGCAGTAC | 338 | TTGCAGGATTAAGGT | 684 | TCCGATTAACCTATC | 753 |
| TAGTTGGGAACGTGG | 297 | TCGCAGTTCGGGAGC | 674 | CCTGCCCGGTACTTA | 753 |
| CCCGCTGAACCGTTT | 291 | AATCCCGGTTACTAA | 667 | TATCACACAAAATGT | 743 |
| ACGTCATTCGGAGAC | 281 | TCTCCCCATACGTAC | 639 | ATGCCTCACCTACCC | 733 |
| TAAATATAACACTGT | 272 | CCTTTGGCGGCGCTT | 624 | AAGGTATGCGTGACA | 733 |
| ACTACGATCTCAAAT | 262 | CTTTAGGGATGTCTT | 596 | GTGAACCGTTCTTAA | 726 |
| AAAAACTGTTTGGTA | 197 | ACTTGTCGTTTAAAG | 574 | CTTCCGTGGCGCTTA | 717 |
| ACAAAATGTGCTGCA | 193 | TTCGTACTGTTAGGC | 573 | CAGTCTGCGGTTGTT | 707 |
| ATACTATCTCTACGA | 190 | CGTGTCACAGGTCCT | 564 | TGACTTGACCTTTGT | 698 |
| CTATTCAGGTTTAGC | 181 | TTTAATGTCTCCCGC | 542 | CTATGAGGACTACCA | 690 |
| AACGGATTTCCCGGA | 145 | CAAGCTCATTCGCAA | 527 | CAAAGACACAAGCGT | 690 |
| AAATCCCATTCTCCC | 122 | TTTCCGGCCTGTTGG | 502 | AGTACCGCCCATTCC | 661 |
| TGCCGTTAATTAATG | 116 | AATCTGACCCGTGGT | 480 | CGATGCGGCATTGTA | 652 |
| CACGCAATAACACAC | 115 | AACAACACTCATAAC | 417 | GAAGTGTGATAGGAA | 646 |
| TTTTACATAGTAGTA | 107 | TACGCGTTTGCGCTC | 414 | AGCCCAAAATCCCTG | 645 |
| TCGGGTGGCGTAATT | 106 | AGCAGTAGTGCACGT | 409 | AGACTCTTCAAGCCC | 639 |
| TATGCATGTTGTTGC | 87 | TTAATGAGGCTGTGT | 406 | ATCAAATCCTCGGCC | 630 |

Table 5 shows the top 30 sequences of and the number of sequences of randomized regions of the nucleic acid after addition of Flag peptide orbiotin and treatment with S1 nuclease. A case where non targe molecule had been added, a case where the Flag peptide had been added, and a case where biotin had been added each exhibited a pattern characteristic to each target molecule. With the use of this method, a nucleic acid aptamer with which a target molecule can be detected and quantified without binding the target molecule to a carrier like beads, can be easily found.

### [Example 7]

Selection for streptavidin using nucleic acid library and sequence analysis of bound nucleic acids: Difference in pattern depending on selection conditions
As a nucleic acid library having modified nucleic acids, the following nucleic acid was used.

5'-cgcctctccgagaccgctgtgtnnnnnnacactctcggagaggcg-3' (SEQ ID NO: 37)
   (where "n" denotes a nucleotide sequence comprising a mixture of g, a, t, and c)

### <Preparation of nucleic acid library and binding to substance>

Each nucleic acid library (2 µl(100 µM)) was added to 200 µl of binding buffer (10 mM Tris-HCl (pH 7.5), 150 mM NaCl, 10 mM MgCl₂, 0.05% Tween 20). After 2-minutes denaturation at 98°C, the mixture was left to stand at 4°C for 1 minute, and then 10 mg/ml ssDNA (as a blocking agent) was added. Subsequently, streptavidin beads or control beads were added. The mixtures were shaken at room temperature for 30 minutes so that aptamers were bound. Washing was then performed 4 times with the binding buffer.

### <Elution procedure>

After washing once with 200 µl of distilled water, the nucleic acid library that had bound to streptavidin beads or control beads was eluted by suspending in 15 µl of distilled water, and then heating at 98°C for 2 minutes.

Meanwhile, elution was also performed under the following conditions in order to examine differences in pattern depending on elution conditions.

The nucleic acid library that had bound to streptavidin was eluted by adding 50 µl of binding buffer and 5 µl of mouse IgG (2.5 mg/ml), flag peptide (10 mM) or streptavidin (2.5 mg/ml) and then shaking for 30 minutes. After elution, phenol chloroform extraction and then ethanol precipitation were performed, and thus the precipitate was dissolved in 15 µl of distilled water.

### <PCR amplification procedure>

The thus eluted nucleic acid aptamer was amplified using the following primer set.

5'-AATGATACGGCGACCACCGAGATCTACACTCTTTCCCTACACGACGCTCTTCCGA
TCT [Bar code sequence] CGCCTCTCCGAGA GTGT-3' (SEQ ID NO: 25 to SEQ ID NO:
28)

5’-CAAGCAGAAGACGGCATACGAGCTCTTCCGATCTCGCCTCTCCGAGACCGCTGT
GT-3’ (SEQ ID NO: 38)

(where [Barcode sequence] is an arbitrary sequence of 4-7 nucleotides used for distinguishing samples upon analysis with a next-generation sequencer. Primers used have barcode sequences that vary from one sample to another.)
PCR was performed using a thermal circular (Biometra), after keeping the temperature at 96°C for 1 minute, for 1 cycle of 95°C for 15 seconds, 46°C for 20 seconds, and 72°C for 60 seconds, then 35 cycles each consisting of 95°C for 15 seconds and 72°C for 60 seconds. The PCR reagent used was KOD-Neo-plus (Toyobo Co., Ltd., Japan).

The amplified PCR product was subjected to sequence analysis using an Illumina Genome Analyzer (Illumina, Inc.) and the DNA primer, 5'-ACACTCTTTCCCTACACGACGCTCTTCCGATCT-3' (SEQ ID NO: 4).

The sequences of the obtained about 10,000,000-to-20,000,000 clusters (i.e., about 10,000,000-to-20,000,000 oligonucleotides) were analyzed. The sequences of the nucleic acid library bound to streptavidin and eluted under each type of conditions were analyzed based on sequences corresponding to the randomized regions of the nucleic acid library added to tubes and barcode sequences indicating the tubes corresponding thereto. Table 6 shows the nucleic acid sequence specifically binding to each substance and the average number of clusters for each of the nucleic acid sequences (the average number of each oligonucleotide detected).

**[Table 6]**

| Sequence of randomized region | Streptavidin beads (Eluting with streptavidin) | Error | Streptavidin beads (Eluting at 98°C) | Error |
|---|---|---|---|---|
| TGCGTC | 522238 | 59554 | 365815 | 6397 |
| TGCGGC | 210523 | 30815 | 282388 | 8297 |
| TGCGCC | 80753 | 8290 | 175901 | 5014 |
| AGCGTC | 7954 | 2472 | 2686 | 568 |
| AAAAAA | 7713 | 5296 | 5389 | 982 |
| AAATAA | 3283 | 3116 | 1515 | 152 |
| AGCGGC | 2873 | 1091 | 2676 | 223 |
| TGCGAC | 2178 | 470 | 11992 | 383 |
| TGCATC | 2031 | 610 | 11632 | 286 |
| CCCCCC | 1960 | 1309 | 757 | 455 |
| AAAAAT | 1905 | 1022 | 1185 | 140 |
| AAAATA | 1661 | 834 | 911 | 143 |
| AGCGCC | 1389 | 460 | 2226 | 311 |
| AAATAT | 1231 | 1271 | 505 | 84 |
| AACAAA | 1135 | 1425 | 510 | 113 |
| TGCGTA | 1096 | 269 | 779 | 78 |
| AAATTA | 1088 | 1095 | 411 | 54 |
| GCCCCC | 988 | 1069 | 266 | 144 |
| AATTAA | 924 | 640 | 376 | 102 |
| AATAAA | 900 | 153 | 1008 | 124 |
| ACCACC | 889 | 1059 | 118 | 60 |
| AAGAAA | 875 | 756 | 599 | 144 |
| CCCCCA | 868 | 1087 | 271 | 121 |
| ACCCCC | 862 | 1099 | 339 | 159 |
| AAGAAT | 840 | 848 | 205 | 64 |
| TAAAAT | 839 | 548 | 430 | 72 |
| AGAAAA | 754 | 825 | 307 | 84 |
| AAATTT | 752 | 566 | 221 | 44 |
| ACAAAT | 728 | 1248 | 56 | 3 |
| TAAAAA | 693 | 673 | 558 | 78 |
| TGCACC | 678 | 314 | 5631 | 451 |

| | | | | |
|---|---|---|---|---|
| (Note) Each value is the average value of 3 samples. | | | | |

Table 6 shows differences in nucleic acid sequence pattern between the case where aptamers bound to streptavidin beads had been eluted after addition of streptavidin and the case where the same had been eluted at 98°C.

These differences were thought to result from a difference in physical binding state, such as Kon or Koff, between an aptamer and a target. Using the obtained information, a substance can be more strictly specified.

### Industrial Applicability

Through the use of a next-generation sequencer and a pool of limited nucleic acids comprising several randomized nucleotides, the detection system based on a completely novel concept is provided, by which the affinity spectra of all nucleic acid aptamers for a target molecule can be obtained exhaustively, and thus the target molecule can be detected and quantified. As a result of establishment of this technique, a system for detecting and quantifying all compounds, proteins, viral types, and the like can be constructed within a short time, and it can serve as an innovative technique in the filed of detection.

All publications, patents, and patent applications cited herein are incorporated herein by reference in their entirety.

### Sequence Listing Free Text

SEQ ID NOS: 1 to 3, 20 to 23, 30 to 31, 36: randomized nucleic acid aptamers
SEQ ID NOS: 1 to 19, 24 to 29, 32 to 35, 37, 38: primers
SEQ ID NO: 39: Flag peptide

## Claims

1. A method for detecting a target molecule, comprising the following steps of:
(a) bringing a target molecule into contact with a collection of oligonucleotides which comprise multiple nucleic acid aptamers having different randomized sequences;
(b) selecting a subcollection of oligonucleotides that bind to the target molecule;
(c) examining the sequence of each oligonucleotide in the selected subcollection;
(d) extracting sequence information which is characteristic to the oligonucleotides having affinity for the target molecule, from the sequences of the selected oligonucleotides; and
(e) identifying the target molecule based on the sequence information.

2. The method according to claim 1, wherein the collection of oligonucleotides has nucleotide sequences represented by general formula X₁N₁X₂N₂...X ₙ₋₁N ₙ₋₁Xₙ (where each X is a specific nucleotide sequence having a specific length, and, at least one X may be deleted from among Xs except for X₁ and Xₙ, each N has a length of 1 nucleotide, and, is G, A, C, T or U, or a modified nucleotide thereof, and "n" is an integer ranging from 2 to 30).

3. The method according to claim 2, wherein X₁ complementarily binds to Xₙ.

4. The method according to any one of claims 1 to 3, wherein the number of nucleic acid aptamers in the collection of oligonucleotides is between 3 and 10⁹, inclusive.

5. The method according to any one of claims 2 to 4, wherein "n" in the general formula is an integer ranging from 5 to 10.

6. The method according to any one of claims 1 to 5, wherein the step (b) of selecting a subcollection of oligonucleotides bound to a target molecule comprises removing a subcollection of oligonucleotides unbound to the target molecule by using the immobilized target molecule or the target molecule bound to a carrier, and then selecting oligonucleotides bound to the target molecule.

7. The method according to any one of claims 1 to 5, wherein the step (b) of selecting a subcollection of oligonucleotides bound to the target molecule comprises digesting unbound oligonucleotides using an enzyme and then selecting oligonucleotides bound to the target molecule.

8. The method according to any one of claims 1 to 7, wherein the step (c) of examining the sequence of each oligonucleotide in the selected subcollection comprises determining the nucleotide sequence.

9. The method according to any one of claims 1 to 8, wherein the step (d) of extracting the sequence information comprises the extraction of sequence information characteristic to the subcolleciton of oligonucleotides having affinity for the target molecule, by comparison of the sequence of subcolleciton of oligonucleotides bind to the target molecule, with the sequence of subcolleciton of oligonucleotides which is obtained with the use of a molecule other than the target or without using any target,

10. The method according to any one of claims 1 to 9, wherein the characteristic subcollection of oligonucleotides obtained by sequencing of claim 8 is used.

11. The method according to any one of claims 1 to 10, further comprising: (f) a step of amplifying the oligonucleotides in the selected subcollection, between the steps (b) and (c).

12. The method according to any one of claims 1 to 11, wherein the sequences of the collection of oligonucleotides partially comprise modified nucleotides.

13. The method according to claim 12, wherein the modification is methylation.

14. The method according to any one of claims 1 to 13, wherein the nucleic acid aptamers in the collection of oligonucleotides are bound to a peptide, an amino acid, a sugar, a polyamine, or a lipid.

15. The method according to any one of claims 1 to 14, wherein the nucleic acid aptamers in the collection of oligonucleotides are chimeras of DNA and RNA.

16. A method for screening for a nucleic acid aptamer specifically binding to a target molecule from the collection of oligonucleotides, **characterized in that** the screening is carried out by using the method according to any one of claims 1 to 15.

17. A method for quantifying a target molecule, comprising: adding a sample containing a target molecule to a nucleic acid aptamer that does not bind to the target molecule, and, a nucleic acid aptamer that specifically binds to the target molecule; treating both nucleic acid aptamers together with nuclease; and then examining the ratio of the amounts of both nucleic acid aptamers thereby to quantify the target molecule.

18. The method according to claim 17, wherein the target molecule is quantified using the nucleic acid aptamer specifically binding to the target molecule, which aptamer is obtained by the method of claim 16.
